# EUROPEAN PATENT APPLICATION

(11) **EP 4 830 770 A1**
(43) Date of publication of application: **28.10.2026**
(21) Application number: 25172209.6
(22) Date of filing: 24.04.2025
(51) Int. Cl.: A61N 2/00, A61N 2/02

(54) **COIL ARRANGEMENT FOR TRANSCRANIAL MAGNETIC STIMULATION AND METHOD FOR PRODUCING THE COIL ARRANGEMENT**

(71) Applicant: Brightmind.AI GmbH, 1010 Wien (AT)
(72) Inventor: Kühne, André, Wien (AT); Kowarik, Gregor, Wien (AT)
(74) Representative: Heeschen Pültz Patentanwälte PartGmbB

(57) **Abstract**

The invention relates to a coil arrangement for transcranial magnetic stimulation, TMS, comprising: a coil stack (100) comprising at least a first coil layer (110) and a second coil layer (120), wherein each of the first and second coil layers (110; 120) comprises an electrically isolating layer and at least one coil (111) with a plurality of windings arranged along the respective coil layer (110; 120), wherein the first coil layer and the second coil layer (110; 120) are stacked in a stacking direction to form the coil stack (100) such that a top surface of the first coil layer (110) is facing a bottom surface of the second coil layer (120), wherein the coil stack (100) is configured to generate a magnetic field for transcranial magnetic stimulation when provided with electric current.

## Description

The invention relates to a coil arrangement for transcranial magnetic simulation, TMS, a device comprising the coil arrangement, a method for producing the coil arrangement and a production system for producing the coil arrangement.

Transcranial magnetic stimulation, TMS, is a non-invasive neuromodulation technique that utilizes time-varying magnetic fields to induce electric currents in targeted regions of the brain. By placing an electromagnetic coil near the scalp, TMS enables the stimulation or inhibition of neuronal activity through electromagnetic induction, without the need for surgical intervention. Originally developed as a diagnostic tool for evaluating motor pathways, TMS has since gained widespread application in both research and clinical settings, including the treatment of neuropsychiatric disorders such as depression, anxiety, and chronic pain. The method's ability to selectively influence brain regions has made it a key modality for exploring brain function and developing therapeutic protocols.

Devices for TMS are thus placed on the head of the patient and specific safety measurements must be provided. In order to generate the magnetic fields, electric current is provided to the device, in particular one or more coils thereof. These coils will generate an amount of heat depending on their shape, structure and arrangement. The depth of the electromagnetic induction is linked to the strength of the magnetic field. In other words, in order to reach a certain depth, a specific strength of the magnetic field is required, which relies on the coils and the electric current provided thereto. Accordingly, a tradeoff between heat dissipation for safety reasons and resulting depth of the magnetic field must be considered when designing such devices.

Devices for TMS use coils manufactured from Litz wire to minimize losses and therefore heat dissipation. Winding coils from Litz wire in more complex geometries than flat, e.g. in cylindrical or spherical shape, is challenging and the manufacturing therefore expensive. Also, devices for TMS with Litz wires suffer from eddy currents when multiple Litz wires are used.

It is an objective of the present invention to provide a coil arrangement for TMS, a device for TMS with such a coil arrangement and a method and production system for producing the coil arrangement which at least improve one or more of the aforementioned drawbacks. In particular, it is an objective of the present invention to provide an improved coil arrangement for TMS being less expensive to manufacture while providing improved results for TMS and improved security.

The objective is solved by a coil arrangement for transcranial magnetic stimulation, TMS, according to a first aspect, the coil arrangement comprising a coil stack comprising at least a first coil layer and a second coil layer. Each of the first and second coil layer comprises an electrically isolating layer and at least one coil. The first coil layer and the second coil layer are stacked in a stacking direction to form the coil stack such that a top surface of the first coil layer is facing a bottom surface of the second coil layer. The coil stack is configured to generate a magnetic field for transcranial magnetic stimulation when provided with electric current.

The inventors have found that by using such coil layers, in particular in the form of PCBs or processed metal sheets, manufacturing costs can be reduced. In addition, each coil layer may be bent into a desired form, in particular a cylindrical geometry with a specific bending radius. Losses due to eddy currents are reduced as compared to bulk conductors because of the layered structure. Each of the coils may comprise or consist of a conductive path, in particular may be made of metal. A coating of the conductive path may not be required since the coil layers may be stacked in a way such that the coils are spaced apart from each other, in particular the electrically isolating layer(s) may be arranged between them and/or the coils are arranged such that they are not stacked directly on each other. As an alternative, the coils of the two coil layers are stacked and thus in contact with each other. Whether the coils of the two coil layers get in contact or not due to the stacking depends on the arrangement or position of the coils and may be based on the use case of the coil arrangement. The user of the coil arrangement may choose more than two coil layers, in particular, a number of coil layers suitable for his/her application. The skilled person will understand from the invention that despite being described for only two coil layers, more than two such coil layers may be implemented and stacked. Accordingly, any such further coil layers may have the same and/or different configuration than the first and/or second coil layer and features described for the first and second coil layers may apply to further coil layers.

One of or more of the coil layers may be a PCB. Accordingly, such a coil layer may have a dielectric substrate layer as the electrically isolating layer and the at least one coil arranged on a top and/or bottom surface of said substrate layer. The at least one coil may be formed by a conductive path comprising or consisting of one or more conductive metals such as cupper or aluminum. In addition, a coating, in particular a lacquer coating may be provided on the coil(s) to electrically isolate at least partially the coil(s). One or more sections of the coil(s) may be spared from the coating to allow an electrical contacting.

One or more of the coil layers may be an electrically conductive metal plate. Via etching methods, laser methods and/or stamping methods, and/or printing methods, the metal plate may form the at least one coil. The metal plate may comprise or consist of copper, silver, zinc or aluminum. A lacquer coating for at least partially electrically isolating the coil may be provided. The lacquer coating may be understood as the electrically isolating layer. In case of aluminum, the at least one coil may be passivated and have a passivation layer, in particular a layer of Aluminum oxide which at least partially covers the coil(s). Also for other materials, a passivation layer might be applied by chemical processing. Said passivation layer may be understood as the electrically isolating layer.

In addition, the form of the conductive path or the electrically conducting material of each coil can be freely chosen. Wires have a predetermined form, namely typically a circular or rectangular cross section, and the user can only choose one diameter for the entire coil. This therefore limits the user regarding flexibility when it comes to choosing the most appropriate forms for optimizing the magnetic field vs. ohmic losses and heat transport. According to the invention, the form of the conductive path of electrically conducting material used for forming the coil can be adapted per layer and may thus vary depending on the intended use and configuration. This flexibility is of even more value when more than one coil is used. In other words, a three-dimensional optimization of the coils is achievable.

Any of the coils of the coil arrangement may comprise or consist of an electrically conducting material, in particular metal. The conductive path may adhere to the isolation layer or the isolating layer may be formed on the conducting material such that a desired shape of the coil itself and/or a desired form of the conductive path itself is provided. In case of the metal sheet, the metal sheet can be processed by known methods to have a desired shape and/or form. The shape and/or form may be based on an TMS application, a magnetic field to be achieved, in particular in a desired target region inside a subject's brain tissue.

The coil stack may be a PCB stack and each of the coil layers may be a respective PCB layer. Such a PCB layer may be a single PCB or a multilayer PCB. The PCB might be of a rigid or a flexible type. The coils of each of the coil layers may adhere to the isolating layer, in particular the dielectric substrate layer. The coil layers may have an identical or different configuration regarding their respective number of coils, forms of the coils and/or positions of the coils. The form of a coil may relate to the overall appearance of the coil as a whole. In addition or as an alternative, the form of the coil may comprise the cross section of the coil. The cross section may be indicative of a width and/or a diameter of the material or conductive path.

At least one or each of the coil layers of the coil stack may have a cylindrical geometry with a predetermined bending radius.

Regarding the PCB layers, the top surface of a coil layer may correspond to a top surface of the dielectric layer of said coil layer and the bottom surface of said coil layer may correspond to a bottom surface of the dielectric layer. The top surface and the bottom surface of the coil layer may be connected by one or more side surfaces. The top and bottom surfaces each may have a substantially greater surface area than the side surface(s).

Regarding the metal sheets, the coil(s) may at least partially or completely form the top and/or bottom surface of the metal sheet.

The coil layers may be stacked such that said coil layers are touching each other and/or are in contact with each other.

The coil layers may be plastic and/or flexible. Plastic means here the ability to be bent to a predetermined bending radius without breaking. Accordingly, the coil layers may be configured to be bent to a desired bending radius or to have a predetermined form without breaking.

The coil stack may further comprise at least one intermediate layer arranged between the first coil layer and the second coil layer in the stacking direction, wherein the intermediate layer is configured to electrically isolate the first coil layer from the second coil layer. The intermediate layer may comprise or consist of dielectric material. Such an intermediate layer may be arranged between any two coil layers of the coil stack. If such an intermediate layer is provided between the first coil layer and the second coil layer, the lacquer layer may be omitted. Similarly, the passivation layer may be omitted, at least where the coil(s) of a coil layer are adhered to an intermediate layer.

The first coil layer and the second coil layer may have at least partially the same orientation, in particular the complete same orientation and/or may be stacked such that the first coil layer and the second coil layer at least partially, in particular fully overlap in the stacking direction.

The first coil layer may comprise a first pair of coils arranged on or forming at least partially the top surface and/or a second pair of coils arranged on or forming at least partially the bottom surface of the isolating layer of the first coil layer, the first pair of coils or the second pair of coils comprising the one coil of the first coil layer. The second coil layer may comprise a third pair of coils arranged on or forming at least partially the top surface and/or a fourth pair of coils arranged on or forming at least partially the bottom surface of the isolating layer of the second coil layer, the third pair of coils or the fourth pair of coils comprising the one coil of the second coil layer. However, the invention is not limited thereto and there may be provided more than two coils per surface. That is, three, four, five, six or more coils may be arranged on the top or bottom surface of the respective isolating layer. Also, more than one pair of coils may be arranged on the top or bottom surface.

Each coil of a coil layer may have a respective plurality of windings along and/or arranged on the respective isolating layer. That is, each of the windings may be arranged on the substrate layer of the PCB layer. Also, at least some or all of the coils of the first coil layer and the second coil layer may be spaced apart from each other. In other words, the stacked coil layers may have their respective coils arranged such that, due to the stacking, the coils of the different coil layers get in contact or not.

The two coils of the first, second, third and/or fourth pair of coils may be arranged such that one coil of the respective pair of coils has a first electrical current flow in one of a clockwise or counter clock wise manner and the other one coil of said respective pair of coils has a second electrical current flow in the other of the clockwise or counter clock wise manner.

The two coils of the first, second, third and/or fourth pair of coils may be formed by a respective single conductive path.

The first pair of coils and the second pair of coils may be arranged on and/or form at least partially the respective surface of the isolating layer such that the first pair of coils and the second pair of coils are at least partially overlapping and/or congruent when viewed along the stacking direction. The third pair of coils and the fourth pair of coils may be arranged on and/or form at least partially the respective surface of the isolating layer such that the third pair of coils and the fourth pair of coils are at least partially overlapping and/or congruent when viewed along the stacking direction.

The conductive paths of the first pair of coils and the second pair of coils may have the same orientation, size and/or form, and/or the conductive paths of the third pair of coils and the fourth pair of coils may have the same orientation, size and/or form.

The first coil layer and the second coil layer may be identical to each other. This means that the first coil layer with its coil or coils is replicated and stacked on the first coil layer. Accordingly, the second coil layer has the exact same configuration as the first coil layer. The coil layers of the coil stack may have all the same configuration.

The first, second, third and/or fourth pair of coils may form a figure-of-eight-coil and/or have a double-d-like form.

Each of the plurality of windings may have an inner end section and an outer end section representing the respective end of the coil. The outer end sections may be connected to each other. In particular, if a pair of coils is formed by a single conductive path, the outer end sections may be merely regarded as theoretical end sections of the respective coil but not as physical end sections. In case each coil is formed by its own conductive path, e.g., each coil of a pair of coils as its own conductive path, the two coils, in particular their conductive paths may be electrically connected by a connecting section connecting the outer end sections of the two coils.

According to another configuration, the first coil layer may have one pair of coils on one of the top or bottom surfaces and either none or one coil on the other one of the top or bottom surfaces. The second coil layer may have one pair of coils on one of the top or bottom surfaces and either none or one coil on the other one of the top or bottom surfaces. Thus, the first coil layer and/or the second coil layer may have different numbers of coils on the respective surfaces.

According to another configuration, the first coil layer may have one coil on one of the top or bottom surfaces and another coil on the other one of the top or bottom surfaces. The second coil layer may have one coil on one of the top or bottom surfaces and another coil on the other one of the top or bottom surfaces. The coils on the top and bottom surfaces may be arranged such that they at least partially overlap and/or are congruent when viewed in the stacking direction.

In case one of the coil layers has only one coil, this coil may be arranged on one of the bottom or top surfaces. This coil may extend at least partially along the respective surface or may completely cover it. In case it only covers partially the respective surface, the coil may be arranged in a center portion. Alternatively, the respective surface may be divided into two halves and the coil may be arranged in one of the halves. As a further alternative, the coil may be arranged on the respective surface based on the TMS application and/or the configuration of the coil arrangement and thus with respect to the other coils of the coil arrangement.

In case one of the coil layers has at least one pair of coils arranged on the same surface, one coil of the pair of coils may be arranged in the first half and the other coil of the pair of coils may be arranged in the second half of this surface. The two coils may be arranged such that the outer end sections both end at a center portion of the isolating layer.

In case one of the coil layers has two coils and the two coils are arranged on different surfaces, the two coils may be arranged such that they do not overlap when viewed in the stacking direction. It is possible to connect the two coils with the connecting section. The connecting section may thus extend from one surface to the other surface of the bottom and top surfaces. Accordingly, the connecting section may extend at least partially through the coil layer.

The coils of the different coil layers may have the same or a different configuration.

Each filament of a coil may be connected to the inner and outer end section of said coil. Furthermore, the filaments do not get in contact with each other along the conductive path. A diameter and/or width of each filament may correspond to a predetermined diameter and/or a predetermined width. Alternatively, the diameter and/or width of at least one of the filaments may vary along the conductive path.

If two coils of a coil layer are connected via a connecting section and the two coils comprise each at least two such filaments, the connection section may comprise at least two section filaments for connecting the at least two filaments of the two coils.

Independently of whether two coils are formed by a single conductive path or are connected via a connection section, when the two coils comprise two filaments, the two filaments may be arranged as follows. The two filaments of such each of these coils may be identified as a first and a second filament. Each of these coils may be winded such that the first and second filament alternate when viewed from an inner portion of the respective coil. At the outer end section, one of the first and second filament may thus be identified as an outer filament and the other one as an inner filament due to the winding. In case of the single conductive path, the outer filament of one of the two coils may be connected to the inner filament of the other of the two coils and the inner filament of the one coil may be connected to the outer filament of the other coil. In other words, the two filaments of the single conductive path run in parallel. In case of the connecting section, the section filaments may either connect the outer filaments with each other and the inner filaments with each other or may connect the outer filaments with the inner filaments of the two coils, the latter resulting in a crossing of the two section filaments.

A distance between neighboring windings of the plurality of windings of at least one of the coils of the first coil layer and the second coil layer may correspond to a predetermined first distance and/or may vary when viewed along the neighboring windings. This may apply to all windings of the plurality of windings. In other words, the distance may increase and decrease at least once or several times, such as two times, three times, and so on, when viewed along the conductive path.

Each of the first coil layer and the second coil layer may further comprise a mounting through hole extending from the bottom surface to the top surface of the respective coil layer, wherein the mounting through hole is arranged in a center portion of the plurality of windings of the respective coil layer. Thus, the plurality of windings may be wound around the respective mounting through hole. The mounting through holes may be configured to receive and/or form a positive fit with a first fixing unit extending in the stacking direction. The fixing unit may be comprised by a housing of a device accosting to a second aspect of the invention. Accordingly, stacking and maintaining the coil layers in a predetermined position is possible.

The first coil layer and the second coil layer may be stacked in the stacking direction such that the mounting through holes are arranged on top of each other in the stacking direction.

Each of the coil layers may have more than one mounting through hole. If two or more mounting through holes are provided, the through holes of each coil layer may be spaced apart from each other. For example, if each coil layer comprises a first mounting through hole and a second mounting through hole, the first through holes and the second through holes may be arranged such that, when the coil layers are stacked, the first through holes are stacked with each other and the second through holes are stacked with each other.

The inner end section of at least one coil of the coil layers and/or the outer end section of at least one coil of the coil layers may comprise an electrical connection elements, in particular a gold finger structure and/or drilled holes coated with conducting material and/or vias in the case of PCBs. The electrical connection element may electrically connect the coil(s) of the top and/or bottom surface. That is, the electrical connection element may be either arranged on one of the top or bottom surface and thus may connect only the coil(s) of the top or bottom surface. As an alternative, the electrical connection element may extend from the top to the bottom surface and may thus connect coil(s) on both sides of such a coil layer. Each coil layer may comprise at least two such electrical connection elements. The gold finger structure and/or via and/or hole with inner coating are suitable for connecting the coil layers to distribute the current density over the entirety of layers (e.g. gold-plated metal reaching the edge of a geometric shape allowing accessing another layer).

Each of the first coil layer and the second coil layer may comprise a cooling through hole extending from the bottom surface to the top surface of the respective coil layer, wherein the cooling through holes are arranged on top of each other in the stacking direction and are configured to conduct a cooling medium therethrough to cool the first coil layer and the second coil layer. The cooling medium may be gaseous or liquid. There may be provided more than one cooling through hole per coil layer.

The coil stack may comprise at least one cooling layer arranged between two neighboring coil layers, wherein the cooling layer comprises at least one cooling channel configured to conduct a cooling medium, the cooling layer being configured to cool the first coil layer and the second coil layer. The cooling channel may thus extend along a direction different to or perpendicular to the stacking direction.

At least one the first coil layer and the second coil layer may further comprise a metallic structure electrically isolated from the coil or the plurality of coils of said coil layer, wherein the metallic structure is configured to absorb heat from the coil or the plurality of coils of said coil layer. The metallic structure may be arranged and/or printed on the same surface as the at least one coil of the coil layer.

The coil stack may comprise at least one temperature stabilizing layer between two coil layers configured to provide a temperature stabilizing effect. The temperature stabilizing effect may be either transporting heat through the use of material with high heat conduction, or by acting as a reservoir using specific heat materials or materials in a condition where the temperature increase from a certain amount of energy is small (e.g. by the use of a phase transition of the material).

The inner end sections of at least one coil of each of the coil layer may have an electrically conductive protrusion protruding from the respective surface the coil is arranged on, wherein the protrusion may be configured to protrude to an upper or lower neighboring coil layer in the stacking direction. The protrusion of said coil layer may be further configured to connect the inner end section of the upper or lower neighboring coil layer. The protrusion may be aligned within the surface of the coil layer, but may be bendable in a different direction than the coil layer's surface the coil is arranged on.

The objective is solved by a device for transcranial magnetic stimulation according to a second aspect, the device comprising a coil arrangement according to first aspect. The device further comprises the housing configured to receive the coil arrangement, the housing having at least one aligning structure configured to align the coil arrangement within the housing.

The objective is solved by a method for producing a coil arrangement for transcranial magnetic stimulation according to a third aspect. The coil arrangement may be a coil arrangement according to the first aspect. The method comprising the steps of:
- providing an aligning structure having a predetermined surface onto which a plurality of coil layers forming a coil stack is stackable, wherein each coil layer extends from a bottom surface to a top surface in a stacking direction;
- providing at least a first coil layer and a second coil layer, each of the first coil layer and the second coil layer comprising an electrically isolating layer and at least one coil with a plurality of windings arranged along the respective coil layer;
- arranging a bottom surface of the first coil layer on the predetermined surface and aligning the first coil layer with the aligning structure;
- stacking the second coil layer on the first coil layer in the stacking direction by arranging a bottom surface of the second coil layer on the top surface of the first coil layer;
- aligning the second coil layer with the aligning structure;
- applying a force on the coil stack such that the top of the first coil layer and the bottom surface of the second coil layers are formed based on the predetermined surface of the aligning structure.

The method may further comprise at least one of:
- prior to arranging the first coil layer on the aligning structure, arranging a foundation layer on the aligning structure and aligning the foundation layer with the aligning structure, the foundation structure being electrically isolating;
- applying to the top surface of the first coil layer an adhesive layer configured to adhere to the top surface of the first coil layer and the bottom surface of the second coil layer;
- arranging a temperature stabilizing sheet between the first and second coil layers;
- after a last coil layer, apply a finishing layer on the top surface of the last coil layer, the finishing layer being electrically isolating;
- electrically connecting, for each coil layer, the coils of the respective coil layer;
- electrically connecting the coils of the different coil layers with each other.

Electrically connecting may comprise contacting using force to ensure proper contact, using solder or a (point) welding technique.

The coil layers may be prepared in advance or may be produced in one or more steps prior to the step of providing the coil layers. The coil layers may be configured as described with reference to the coil arrangement according to the first aspect.

The objective is solved by a production system for producing a coil arrangement for transcranial magnetic stimulation according to a fourth aspect, comprising means for carrying out the method according to the third aspect.

Features described with reference to the coil arrangement according to the first aspect may also be implemented as features of the method according to the third aspect and the production system of the fourth aspect and vice versa.

The objective is solved by a computer program product according to a fifth aspect, comprising instructions which, when the program is executed by a production system according to fourth aspect, cause the production system to carry out the method of the third aspect.

Further details, advantages and aspects of the present disclosure will become apparent from the following embodiments taken in conjunction with the drawings, wherein:
- Figs. 1a) and b): show a first and second embodiment of a coil arrangement;
- Fig. 2: shows a top surface of a coil layer of the coil arrangement;
- Fig. 3: shows a device for TMS with such a coil arrangement;
- Fig. 4: shows a method for producing a coil arrangement; and
- Fig. 5: shows a production system for producing a coil arrangement.

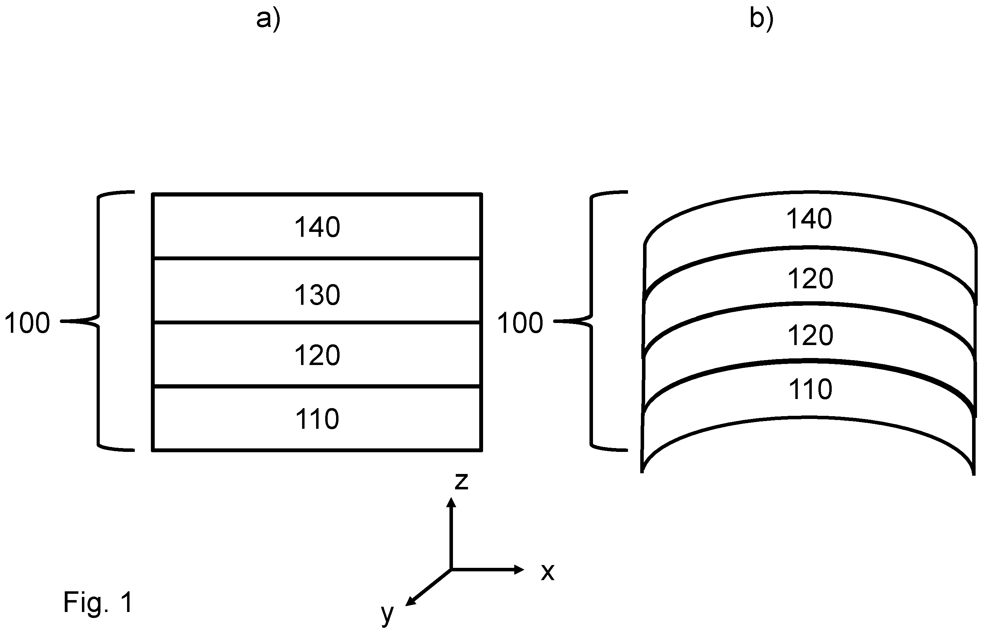

Figs. 1 a) and b) show two different embodiments of a coil arrangement for TMS. Each of the coil arrangements shown in Fig. 1 comprise a coil stack 100 comprising a first coil layer 110, a second coil layer 120, a third coil layer 130 and a fourth layer 140. Each of the coil layers 110-140 comprises an electrically isolating layer, here a dielectric substrate layer onto which at least one coil is adhering to. That is, the dielectric substrate layer has a top and a bottom surface, which are connected by at least one side surface. Each coil may consist of one or more metals, in particular without having any kind of isolation sheath or isolating coating. The coil layers 110-140 are stacked such that the top surface of the first coil layer 110 faces a bottom surface of the second coil layer 120, a top surface of the second coil layer 120 faces a bottom surface of the third coil layer 130 and a top surface of the third coil layer 130 faces a bottom surface of the fourth coil layer 140.

The coil layers 110-140 are stacked in a stacking direction, which is parallel to the z-axis of Fig. 1. The at least one coil of each coil layer 110-140 is printed on the top or bottom surface. If any of the coil layers 110-140 comprises more than two coils, at least two coils may be adhering to the top surface or the bottom surface or at least one coil may be adhering to the top surface and at least one to the bottom surface.

The coil stack 100, in particular the coil layers 110-140 may be plastic allowing them to be bent to a desired form. In addition or as an alternative, the coil layers 110-140 may be elastic and may thus be bent, e.g., by a user. That is, Fig. 1 a) shows the coil arrangement with the coil stack 100 consisting of planar coil layers 110-140, while Fig. 1 b) shows the coil arrangement with a predetermined bending radius. The coil layers 110-140 may be PCBs. PCBs have the advantage that they can be relatively cheap in production costs while also allowing a high degree of accuracy of the printed elements thereof. Furthermore, the printed elements can have arbitrary forms allowing maximum flexibility when it comes to design choices. According to the present invention, the coils of the coil layers 110-140 comprise or consist of an electrically conductive material or multiple of such materials, which may be printed on the respective surface. This means that the design of the coils can be flexibly chosen to meet the requirements of the TMS application, e.g., a desired depth of a magnetic fields generated by the coils.

To provide an even more simplified production of the coil layers 110-140, the coils of the coil layers are spaced apart from each other, also when they are stacked. Due to the dielectric substrate layer and the apart spacing, further electrically isolating materials may be omitted. This may lead to designs of coils wherein neighboring windings are relatively close to each other without being in contact. Compared to the use of Litz wires, Litz wires have a predetermined cross section. Also, the diameter of such a wire is predetermined and cannot be changed for a single coil. In addition, Litz wires comprise the conducting material as well as at least one isolating sheath, thereby increasing the diameter of the cross section. This automatically leads to less densely arranged windings.

The coil stack 100 is configured to generate a magnetic field for transcranial magnetic stimulation when provided with electric current. In particular, the current is provided to the coils of the coil layers 110-140 which thereby output a respective magnetic field. By exploiting the flexible design choices of PCBs and/or other techniques of stacking isolating and conductive layers, it is possible to provide coil arrangements for TMS with high accuracy, cheap in production and an improved flexibility regarding the design of each coil. Regarding the design of each coil, not only the overall form of the coils itself may be chosen with improved flexibility, but also within a single coil, parameters thereof such as a width along the X-Y-axis or a height along the z-Axis (in Fig. 1) may vary. In other words, along the conductive path of a single coil, the cross section, the width and/or the height may vary. Also, a distance between two neighboring windings may vary along the conductive path of the single coil.

This improved design flexibility offers the possibility to account for, e.g., eddy currents, heat generation and/or dissipation, positive and/or negative interference of the magnetic fields as well as an overall design of the coil arrangement. Most TMS applications desire little to no distance between the TMS device, in particular the coils thereof and the skin of the head of the patient. Due to the individually formed heads, it may be challenging to provide one design for all individuals. Moreover, the coil arrangement, in particular with PCB layers 110-140 is cost efficient on scale.

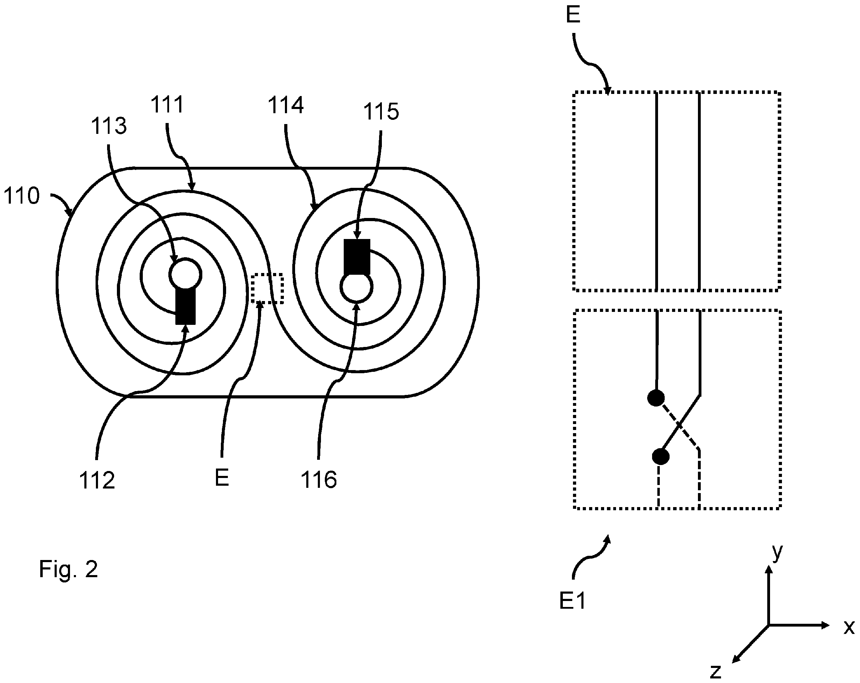

As shown in Fig. 2, the first coil layer 110 has two coils 111, 114 on the top surface of the isolation layer thereby forming a first pair of coils. The other coil layers 120-140 may be configured identically to the first coil layer 110. The invention is not limited thereto and three or more coils per surface may be provided. While the positions of the coils 111, 114 in Fig. 2 show a preferred embodiment, the invention is not limited thereto and different positions may be chosen, in particular based on the TMS application. Furthermore, the form of the coils 111, 114 shown in Fig. 2 is merely one example, but the invention is not limited thereto. It may be preferred that each coil layer 110-140 has the same configuration regarding the number of the coils, the position of the coils, the form of the coils and/or the form of the conductive paths of the respective coil. Thus, the coil layers 110-140 may have a uniform configuration.

As shown in Fig. 2, the two coils 111, 114 each have a plurality of windings. Moreover, both coils 111, 114 are formed by a single conductive path which starts at a first electrical connection element 112 and is wound around said first electrical connection element 112.

Here, the conductive path is wound in a clock wise manner. Then, in order to form the coil 114, the conductive path is wound in a counter clock wise manner until it ends at a second electrical connection element 112. The electrical connection elements 112, 115 may serve as an input and output for electrical current for the coils 111, 114. Said first and second electrical connection elements 112, 115 may be goldfinger contacts possibly including vias and/or holes with a conductive coating.

Furthermore, the coil layer 110 comprises two mounting through holes 113, 116. The mounting through holes 113, 116 extend in the stacking direction, here parallel to the z-axis. In order to stack the plurality of coil layers 110-1140 of the coil stack 100 and to help them staying aligned, each of said coil layers 110-140 may comprise at least one such mounting through hole. The through holes of each coil layer 110-140 may have the same position, allowing to input an aligning structure after stacking the coil layers 110-140 into and through the mounting through holes. The aligning structure may be rod-shaped or may have a cylindrical form.

The conductive path may comprise or consist of two or more electrically conductive filaments. Fig. 2 shows this configuration via the zoomed dotted box E. As illustrated in the dotted box E in Fig. 2, the conductive path consists of two filaments. Said two filaments are arranged parallel to each other and/or a spaced apart from each other with a predetermined distance. The two filaments may be defined as a pair or bundle, in particular in case of three or more filaments. A distance between individual A distance between two neighboring windings of the conductive path and along the conductive path may correspond to a second distance or may vary along the conductive path. In a preferred embodiments, the distance between the filaments of a bundle may be smaller than a distance between the neighboring windings of the conductive path. Depending on the geometry of the magnetic field for the application, it can be favorable to introduce a crossing between the filaments in the central connecting section of two coils, to cancel out the driving electro-motoric forces and hence reduce losses. These crossings of filaments are illustrated in Fig. 2 zoomed box E1, indicating the conductor from one side (solid lines) of the isolator to the other side (dashed lines) as black dots.

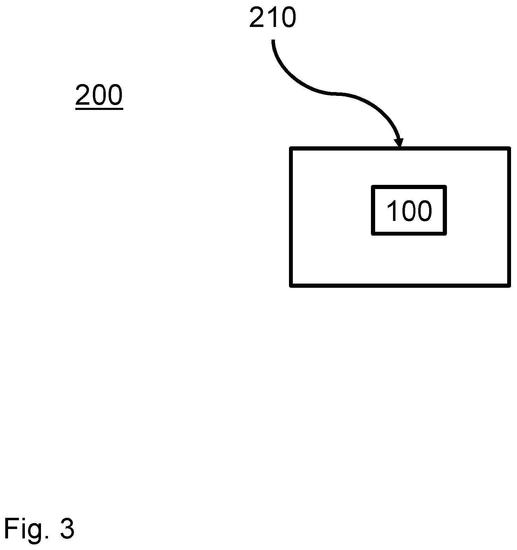

Fig. 3 shows a device 200 for transcranial magnetic stimulation, comprising a coil arrangement 100, e.g. the one of Fig. 1 a) or b) and a housing 210 configured to receive the coil arrangement 100. The housing 100 has at least one aligning structure configured to align the coil arrangement within the housing. The aligning structure may comprise means suitable to being inserted into the mounting through holes. Also, the housing may have a form based on the form of the coil stack 100. That is, the housing may comprise a bottom surface, a top surface and at least one side surface connecting the bottom and top surface. At least one of the bottom and top surface may correspond to the bottom or top surface of the coil layers 110-140 such that the coil stack 100 can be arranged on the bottom surface of the housing 210. In particular, the bottom surface of the housing 210 may be configured such that the bottom surface of the first coil layer 110 may be at least partially, in particular completely rest on the bottom surface of the housing 210. The at least one side surface may limit the bottom and/or top surface of the housing 210 such that the at least one side surface aligns the coil stack 100 inside the housing 210.

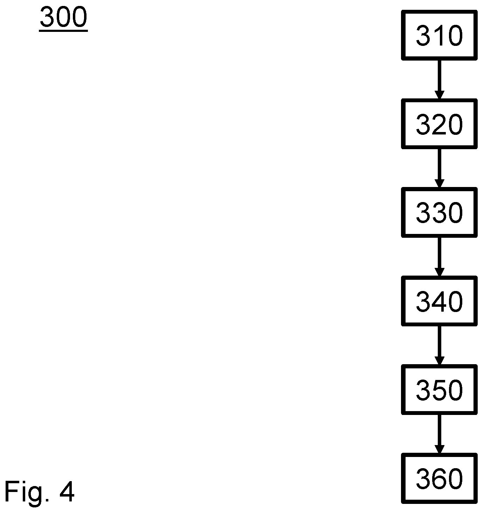

Fig. 4 shows a method 300 for producing a coil arrangement for transcranial magnetic stimulation as, e.g., illustrated in Fig. 1a) or b), comprising the steps of:
- providing 310 an aligning structure having a predetermined surface onto which a plurality of coil layers forming a coil stack 100 is stackable, wherein each coil layer 110-140 extends from a bottom surface to a top surface in a stacking direction;
- providing 320 at least a first coil layer 110 and a second coil layer 120, each of the first coil layer 110 and the second coil layer 120 comprising an electrically isolating layer and at least one coil with a plurality of windings arranged along the respective coil layer;
- arranging 330 a bottom surface of the first coil layer 110 on the predetermined surface and aligning the first coil layer 110 with the aligning structure;
- stacking 340 the second coil layer 120 on the first coil layer 110 in the stacking direction by arranging a bottom surface of the second coil layer 120 on the top surface of the first coil layer 110;
- aligning 350 the second coil layer 120 with the aligning structure;
- applying 360 a force on the coil stack 100 such that the top of the first coil layer 110 and the bottom surface of the second coil layer 120 are formed based on the predetermined surface of the aligning structure.

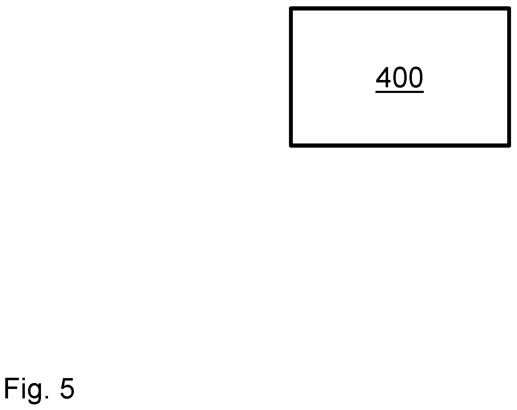

The method 300 may be stored in the form of a computer program product, e.g., on storage means of a production system 400. Fig. 5 shows the production means 400 comprising means for carrying out the method 300.

Fig. 5 shows a production system 400 configured to execute the method 300. The production system 400 may comprise respective means for carrying out the method 300.

### REFERENCE SIGNS

- 100: coil stack
- 110: first coil layer
- 111: first coil
- 112: first electrical connection element
- 113: first mounting through hole
- 114: second coil
- 115: second electrical connection element
- 116: second mounting through hole
- 120: second coil layer
- 130: third coil layer
- 140: fourth coil layer
- 200: Device for transcranial magnetic stimulation
- 210: housing
- 300: method for producing a coil arrangement
- 310: providing an aligning structure
- 320: providing at least a first coil layer and a second coil layer
- 330: arranging a bottom surface of the first coil layer on the predetermined surface
- 340: stacking the second coil layer on the first coil layer
- 350: aligning the second coil layer with the aligning structure
- 360: applying a force on the coil stack
- 400: production system for producing a stack arrangement

## Claims

1. Coil arrangement for transcranial magnetic stimulation, TMS, comprising:
a coil stack (100) comprising at least a first coil layer (110) and a second coil layer (120),
wherein each of the first and second coil layers (110; 120) comprises an electrically isolating layer and at least one coil (111) with a plurality of windings arranged along the respective coil layer (110; 120),
wherein the first coil layer and the second coil layer (110; 120) are stacked in a stacking direction to form the coil stack (100) such that a top surface of the first coil layer (110) is facing a bottom surface of the second coil layer (120),
wherein the coil stack (100) is configured to generate a magnetic field for transcranial magnetic stimulation when provided with electric current.

2. Coil arrangement according to any of the previous claims,
wherein the first coil layer (110) comprises a first pair of coils (111; 114) arranged on or forming at least partially the top surface and/or a second pair of coils arranged on or forming at least partially the bottom surface of the isolating layer of the first coil layer (110), the first pair of coils or the second pair of coils comprising the one coil (111) of the first coil layer (110), and/or
wherein the second coil layer (120) comprises a third pair of coils arranged on or forming at least partially the top surface and/or a fourth pair of coils arranged on or forming at least partially the bottom surface of the isolating layer of the second coil layer (120), the third pair of coils or the fourth pair of coils comprising the one coil of the second coil layer (110) ,
wherein each of the coils (111; 114) of the first coil layer and the second coil layer (110; 120) has a plurality of windings along the respective top or bottom surface.

3. Coil arrangement according to claim 2,
wherein the two coils (111; 114) of the first, second, third and/or fourth pair of coils are arranged such that one coil of the respective pair of coils has a first electrical current flow in one of a clockwise or counter clock wise manner and the other one coil of said respective pair of coils has a second electrical current flow in the other of the clockwise or counter clock wise manner.

4. Coil arrangement according to claim 2 or 3,
wherein the two coils (111; 114) of the first, second, third and/or fourth pair of coils are formed by a respective single conductive path.

5. Coil arrangement according to any of claims 2 to 4,
wherein the first pair of coils (111; 114) and the second pair of coils are arranged on or forming at least partially the respective surface of the coil layer (110; 120) such that the first pair of coils and the second pair of coils are at least partially overlapping and/or congruent when viewed in the stacking direction, and/or
wherein the third pair of coils and the fourth pair of coils are arranged on or forming at least partially the respective surface of the coil layer such that the third pair of coils and the fourth pair of coils are at least partially overlapping and/or congruent when viewed in the stacking direction.

6. Coil arrangement according to any of claims 2 to 5,
wherein the conductive paths of the first pair of coils (111; 114) and the second pair of coils have the same orientation, size and/or form, and/or
wherein the conductive paths of the third pair of coils and the fourth pair of coils have the same orientation, size and/or form.

7. Coil arrangement according to any of the previous claims,
wherein each coil (111; 114) of the first coil layer and the second coil layer (110; 120) has its conductive path formed by the respective plurality of windings,
wherein the conductive path comprises two electrically conductive filaments extending along the conductive path,
wherein the two filaments are spaced apart from each other.

8. Coil arrangement according to any of the previous claims,
wherein a distance between neighboring windings of the plurality of windings of at least one of the coils (111; 114) of the first coil layer and the second coil layer (110; 120) corresponds to a predetermined first distance and/or is varying when viewed along the neighboring windings.

9. Coil arrangement according to any of the previous claims,
wherein each of the first coil layer and the second coil layer (110; 120) further comprises a mounting through hole (113; 116) extending from the bottom surface to the top surface of the respective coil layer (110; 120),
wherein the mounting through hole (113; 116) is arranged in a center portion of the plurality of windings of the respective coil layer (110; 120),
wherein the mounting through holes (113; 116) are configured to receive and/or form a positive fit with a first fixing unit extending in the stacking direction.

10. Coil arrangement according to any of the previous claims,
wherein each of the first coil layer and the second coil layer (110; 120) comprises a cooling through hole extending from the bottom surface to the top surface of the respective coil layer (110; 120),
wherein the cooling through holes are arranged on top of each other in the stacking direction and are configured to conduct a cooling medium therethrough to cool the first coil layer and the second coil layer (110; 120).

11. Coil arrangement according to any of the previous claims,
wherein the coil stack (100) comprises at least one cooling layer arranged between the first coil layer and the second coil layer (110; 120),
wherein the cooling layer comprises at least one cooling channel configured to conduct a cooling medium, the cooling layer being configured to cool the first coil layer and the second coil layer (110; 120).

12. Coil arrangement according to any of the previous claims,
wherein at least one the first coil layer and the second coil layer (110; 120) further comprises a metallic structure electrically isolated from the coil or the coils (111; 114) of the plurality of coils (111; 114) of said coil layer (110; 120),
wherein the metallic structure is configured to absorb heat from the coil (111) or the coils (111; 114) of said coil layer (110; 120).

13. Device (200) for transcranial magnetic stimulation, comprising a coil arrangement according to any of the previous claims, further comprising:
a housing (210) configured to receive the coil arrangement, the housing (210) having at least one aligning structure configured to align the coil arrangement within the housing (210).

14. Method (300) for producing a coil arrangement for transcranial magnetic stimulation, comprising the steps of:
providing (310) an aligning structure having a predetermined surface onto which a plurality of coil layers (110; 120) forming a coil stack (110) is stackable, wherein each coil layer (110; 120) extends from a bottom surface to a top surface in a stacking direction;
providing (320) at least a first coil layer (110) and a second coil layer (120), each of the first coil layer (110) and the second coil layer (120) comprising an electrically isolating layer and at least one coil (111) with a plurality of windings arranged along the respective coil layer (110; 120);
arranging (330) a bottom surface of the first coil layer (110) on the predetermined surface and aligning the first coil layer (110) with the aligning structure;
stacking (340) the second coil layer (120) on the first coil layer (110) in the stacking direction by arranging the bottom surface of the second coil layer (120) on the top surface of the first coil layer (110);
aligning (350) the second coil layer (120) with the aligning structure;
applying (360) a force on the coil stack (100) such that the top of the first coil layer (110) and the bottom surface of the second coil layer (120) are formed based on the predetermined surface of the aligning structure.

15. Production system (400) for producing a coil arrangement for transcranial magnetic stimulation, comprising means for carrying out the method (100) according to claim 14.

16. Computer program product, comprising instructions which, when the program is executed by a production system (400) according to claim 15, cause the production system (400) to carry out the method (100) of claim 14.
